# EUROPÄISCHE PATENTANMELDUNG

(11) **EP 4 606 782 A1**
(43) Veröffentlichungstag der Anmeldung: **27.08.2025**
(21) Anmeldenummer: 24020064.2
(22) Anmeldetag: 23.02.2024
(51) Int. Cl.: C07C 29/151, C07C 29/80, C07C 31/04

(54) **VERFAHREN UND ANLAGE ZUR HERSTELLUNG EINES METHANOLPRODUKTS**

(71) Anmelder: Linde GmbH, 82049 Pullach (DE); Technische Universität München, 80333 München (DE)
(72) Erfinder: Dillig, Marius, 82049 Pullach (DE); Haselsteiner, Thomas, 82049 Pullach (DE); Klein, Harald, 80290 München (DE); Fahr, Steffan, 80290 München (DE)
(74) Vertreter: Fischer, Werner

(57) **Zusammenfassung**

Es wird ein Verfahren (100) zur Erzeugung eines Methanolprodukts (20), vorgeschlagen, das ein Bereitstellen von Methanol und Wasser enthaltendem Rohmethanol (12), ein Bereitstellen eines Methanolzwischenprodukts (16) mit einem über die Zeit schwankenden Methanolgehalt unter Verwendung des Rohmethanols (12), ein Einspeisen des Methanolzwischenprodukts (16) in eine Tankanordnung (B), und ein Bereitstellen des Methanolprodukts (20) umfassend eine Entnahme des in der Tankanordnung (B) gespeicherten Methanolzwischenprodukts (16) umfasst, wobei die Entnahme des in der Tankanordnung (B) gespeicherten Methanolzwischenprodukts (16) so gesteuert wird, dass das Methanolprodukt (20) einen Methanolgehalt in einem vorgegebenen Bereich aufweist. Eine entsprechende Anlage wird ebenfalls vorgeschlagen.

## Beschreibung

Die Erfindung betrifft ein Verfahren und eine Anlage zur Herstellung eines Methanolprodukts.

### Hintergrund

Die Methanolherstellung ist ein Schlüsselprozess in der chemischen Industrie. Methanol stellt eine wichtige Grundchemikalie für die Herstellung von beispielsweise Formaldehyd, Acetaten und Kunststoffen dar und kann zudem als Treibstoffzusatz verwendet werden. Aufgrund seiner effizeineten Umsatzbarkeit wird derzeit hauptsächlich Erdgas als Ausgangsmaterial für die Methanolsynthese eingesetzt. Alternativ kann aber auch Kohle oder Biomasse verwendet werden.

Im Falle der Nutzung von Erdgas wird dieses typischerweise zunächst einer Dampfreformierung unterzogen, um Synthesegas, eine Mischung aus Wasserstoff sowie Kohlenstoffmonoxid und/oder Kohlenstoffdioxid, zu erzeugen. Dieser Schritt beinhaltet die Reaktion von Erdgas mit Wasserdampf bei hohen Temperaturen und unter Verwendung eines Katalysators. Bei der Verwendung von Kohle oder Biomasse kommt oft die partielle Oxidation zum Einsatz.

Vor seinem Einsatz in der Methanolsynthese muss das erzeugte Synthesegasgereinigt werden, um Verunreinigungen wie Schwefelverbindungen zu entfernen, die den Katalysator für die Methanolsynthese schädigen oder deaktivieren könnten. Das gereinigte Synthesegas wird dann unter hohem Druck (5 bis 10 MPa) und bei moderaten Temperaturen (200 bis 300 °C) umgesetzt. Katalysatoren auf Basis von Kupfer, Zinkoxid und Aluminiumoxid sind hierbei üblich. Die Reaktion erfolgt typischerweise in einem Festbettreaktor.

Das Produkt aus der Methanolsynthese enthält neben Methanol auch Wasser und höhere Alkohole. Durch Destillation wird hieraus reines Methanol abgetrennt. Dieser Schritt kann mehrstufig durchgeführt werden, um die gewünschten Reinheiten zu erreichen. Das gereinigte Methanol wird gelagert und als Produkt verkauft oder als Ausgangsstoff für die Herstellung anderer Chemikalien verwendet.

Zukünftige Entwicklungen werden zunehmend auf erneuerbare Rohstoffe und nachhaltigere Produktionsmethoden abzielen. Eine Methanolsynthese aus Elektrolysewasserstoff und rückgewonnenem Kohlenstoffdioxid ist dabei möglich, bringt aber bestimmte Herausforderungen mit sich.

### Überblick

Vorliegend werden eine Anlage und ein Verfahren zur Herstellung von Methanol mit den Merkmalen der unabhängigen Patentansprüche vorgeschlagen. Vorteilhafte Ausgestaltungen und Weiterbildungen sind Gegenstand der abhängigen Patentansprüche sowie der nachfolgenden Beschreibung.

Das vorgeschlagene Verfahren zur Herstellung von Methanol umfasst ein Bereitstellen von Methanol und Wasser enthaltendem Rohmethanol, ein Bereitstellen eines gegenüber dem Rohmethanol an Methanol angereicherten und Wasser abgereicherten Methanolzwischenprodukts mit einem über die Zeit schwankenden Methanolgehalt unter Verwendung des Rohmethanols oder eines Teils hiervon, ein Einspeisen des Methanolzwischenprodukts oder eines Teils hiervon in eine Tankanordnung, und ein Bereitstellen des Methanolprodukts, umfassend eine Entnahme des in der Tankanordnung gespeicherten Methanolzwischenprodukts oder eines Teils hiervon, wobei die Entnahme des in der Tankanordnung gespeicherten Methanolzwischenprodukts derart vorgenommen wird, dass das Methanolprodukt einen Methanolgehalt in einem Vorgabebereich aufweist.

Das hier vorgeschlagene Verfahren und seine Ausgestaltungen ermöglichen eine Reduzierung des Energieverbrauchs für die Aufarbeitung des Rohmethanols, die zu Lasten einer Reinheit des Aufbereitungsprodukts, hier als Methanolvorprodukt bezeichnet, geht. Durch eine Zwischenspeicherung und die Bereitstellung des Methanolvorprodukts zeitweise in einer höheren als der erforderlichen Reinheit und zeitweise einer geringeren als der erforderlichen Reinheit kann dennoch ein Methanolprodukt mit der erforderlichen Reinheit bereitgestellt werden. Das vorgeschlagene Verfahren ist damit in besonders vorteilhafter Weise an die Anforderungen bei der direkten Hydrierung von Kohlenstoffdioxid zu Methanol, beispielsweise unter Einsatz von Elektrolysewasserstoff und/oder Kohlenstoffdioxid aus einer Rauchgasabtrennung, anpassbar.

In einer Ausgestaltung des vorgeschlagenen Verfahrens kann die Entnahme des in der Tankanordnung gespeicherten Methanolzwischenprodukts dann vorgenommen werden, wenn sich in der Tankanordnung aufgrund der Einspeisung ein Methanolgehalt in dem Vorgabebereich eingestellt hat. Entsprechende Ausgestaltungen kommen dabei prinzipiell mit einem Tank aus. Die Einspeisung des Methanolvorprodukts mit einer zeitweise höheren als der erforderlichen Reinheit und zeitweise einer geringeren als der erforderlichen Reinheit führt dabei nach Art einer Mittelwertbildung zu einem bestimmten Zeitpunkt dazu, dass das gespeicherte Methanol die erforderliche Reinheit aufweist.

In einer Ausgestaltung des vorgeschlagenen Verfahrens kann eine Tankanordnung mit mehreren Methanolzwischenprodukttanks verwendet werden, wobei das Methanolzwischenprodukt in Abhängigkeit von seinem Methanolgehalt in unterschiedliche der Methanolzwischenprodukttanks eingespeist wird. Hierbei können gezielt Tanks zur Speicherung des Methanolvorprodukts mit höherer und geringerer Reinheit vorgesehen sein, was insbesondere dann von Vorteil ist, wenn eine künftige Entwicklung der Energieverfügbarkeit nicht vorhersehbar ist.

In einer Ausgestaltung des vorgeschlagenen Verfahrens kann das Bereitstellen des Methanolprodukts eine Entnahme des in der Tankanordnung gespeicherten Methanolzwischenprodukts aus einem oder mehrerer der unterschiedlichen Methanolzwischenprodukttanks umfassen. Das Methanolprodukt kann dabei vorteilhafterweise durch Vermischen entsprechender Anteile bereitgestellt werden. Es ist auch möglich, separate Methanolprodukte mit unterschiedlichen Reinheiten bereitzustellen und deren Reinheit beispielsweise jeweils wie für einen einzigen Methanolzwischenprodukttank erläutert einzustellen. Auch eine Zuordnung von Tanks zu bestimmten erwarteten Reinheiten usw. ist möglich.

In einer Ausgestaltung des vorgeschlagenen Verfahrens kann das Bereitstellen des Methanolzwischenprodukts unter Verwendung einer Destillationssäule vorgenommen werden. Insbesondere kann in dem vorgeschlagenen Verfahren die Destillationssäule die einzige verwendete Destillationssäule sein.

In einer Ausgestaltung des vorgeschlagenen Verfahrens kann die Destillationssäule einen Sumpfverdampfer aufweisen, der mit einer über die Zeit schwankenden Heizleistung betrieben wird. Hierdurch erfolgt zumindest zum Teil die Anpassung an die Energieverfügbarkeit und damit an die spezifischen Anforderungen bei der Herstellung von Methanol aus den erwähnten Ausgangsprodukten.

In einer Ausgestaltung des vorgeschlagenen Verfahrens kann die Heizleistung an eine Energieverfügbarkeit und/oder einen Energiepreis angepasst werden. Eine Energieverfügbarkeit kann dabei auch eine Verfügbarkeit von Wärmeträgern wie Dampf aus anderen Verfahrensschritten sein, beispielsweise Abwärme der Methanolsynthese in einem entsprechenden Reaktor.

In einer Ausgestaltung des vorgeschlagenen Verfahrens kann das Rohmethanol einen Gehalt von 45 bis 55 mol-% Methanol, von 45 bis 55 mol-% Wasser und von 0 bis 2 mol-% weiterer Komponenten aufweisen. Insbesondere kann das Rohmethanol frei oder im Wesentlichen frei an störenden Komponenten wie insbesondere Schwefelverbindungen sein, wie dies insbesondere bei der Verwendung von Wasserstoff und Kohlenstoffdioxid großer Reinheit der Fall ist.

In einer Ausgestaltung des vorgeschlagenen Verfahrens kann das Methanolvorprodukt mit einem Gehalt von 80 bis 100% Methanol auf molarer, Masse- oder Volumenbasis bereitgestellt werden und der Methanolgehalt des Methanolprodukts bei 90 bis 100% oder 95 bis 98% auf molarer, Masse- oder Volumenbasis liegen. Entsprechende Werte ergeben sich ebenfalls insbesondere bei der Umsetzung von Wasserstoff und Kohlenstoffdioxid aus den mehrfach erläuterten Quellen. In einer Ausgestaltung des vorgeschlagenen Verfahrens kann das Bereitstellen des Rohmethanols daher ein Umsetzen von Wasserstoff mit Kohlenstoffdioxid umfassen.

Der Lastbereich, in dem die Einrichtung zur Aufarbeitung des Methanolrohprodukt betrieben werden kann, entspricht aus technischen Gründen nicht notwendigerweise dem Lastbereich, bei dem die Methanolsynthese betrieben werden kann. Insbesondere kann Aufarbeitung eine höhere Mindestauslastung aufweisen. In einer Ausgestaltung des vorgeschlagenen Verfahrens kann das Rohmethanol in einem Rohmethanolspeicher zwischengespeichert werden. Auf diese Weise lässt sich eine Aufbereitung des Rohmethanols weiterhin durchführen, beispielsweise unter Verwendung einer Destillationssäule, die bei geringer Last betrieben wird, während die Methanolsynthese bei einer Teillast arbeitet, die für die Destillationssäule ansonsten ggf. nicht erreichbar ist.

In einer Ausgestaltung des vorgeschlagenen Verfahrens kann ein modellbasiertes Regelungsverfahren verwendet werden, beispielsweise um eine künftige Verfügbarkeit von Energie zu prognostizieren und/oder die Trenneigenschaften der Destillationssäule bei unterschiedlichen Reboilerleistungen zu modellieren.

In einer Ausgestaltung des vorgeschlagenen Verfahrens kann Prozesswasser mit einem über die Zeit unterschiedlichen Gehalt von anderen Komponenten bereitgestellt und zeitweise zwischengespeichert werden. Hierdurch braucht eine Destillationssäule beispielsweise nicht ständig derart betrieben werden, dass das hier abgetrennte Prozesswasser eine Reinheitsanforderung hinsichtlich der zulässigen Gehalte im Abwasser erfüllt. Vielmehr kann auch hier ein Mittelwert eingestellt werden, wie zuvor unter Bezugnahme auf das Methanolvorprodukt erläutert.

Die vorgeschlagene Anlage zur Erzeugung eines Methanolprodukts ist zum Bereitstellen von Methanol und Wasser enthaltendem Rohmethanol, zum Bereitstellen eines gegenüber dem Rohmethanol an Methanol angereicherten und Wasser abgereicherten Methanolzwischenprodukts mit einem über die Zeit schwankenden Methanolgehalt unter Verwendung des Rohmethanols oder eines Teils hiervon, zum Einspeisen des Methanolzwischenprodukts oder eines Teils hiervon in eine Tankanordnung, und zum Bereitstellen des Methanolprodukts umfassend eine Entnahme des in der Tankanordnung gespeicherten Methanolzwischenprodukts oder eines Teils hiervon eingerichtet, wobei die Anlage ferner dazu eingerichtet ist, die Entnahme des in der Tankanordnung gespeicherten Methanolzwischenprodukts derart vorzunehmen, dass das Methanolprodukt einen Methanolgehalt in einem Vorgabebereich aufweist.

Zu weiteren Merkmalen und Vorteilen einer entsprechenden Anlage und Ausgestaltungen hiervon sei auf die obigen Erläuterungen betreffend das vorgeschlagene Verfahren und seine Ausgestaltungen ausdrücklich verwiesen, da diese hierfür in gleicher Weise gelten.

Entsprechendes gilt auch für eine Anlage, die dazu eingerichtet sein kann, ein Verfahren gemäß einer beliebigen Ausgestaltung durchzuführen.

### Zeichnungen

Ausführungsformen der Erfindung werden nachfolgend rein beispielhaft unter Bezugnahme auf die beigefügte Zeichnung beschrieben, wobei
Figur 1 Aspekte einer Methanolsynthese zeigt; und
Figur 2 Eigenschaften von Destillationskolonnen zur Methanolreinigung zeigt.

### Ausführungsformen

Die nachfolgend beschriebenen Ausführungsformen werden lediglich zu dem Zweck beschrieben, den Leser beim Verständnis der beanspruchten und zuvor erläuterten Merkmale zu unterstützen. Sie stellen lediglich repräsentative Beispiele dar und sollen hinsichtlich der Merkmale der Erfindung nicht abschließend und/oder beschränkend betrachtet werden. Es versteht sich, dass die zuvor und nachfolgend beschriebenen Vorteile, Ausführungsformen, Beispiele, Funktionen, Merkmale, Strukturen und/oder anderen Aspekte nicht als Beschränkungen des Umfangs der Erfindung, wie er in den Ansprüchen definiert ist, oder als Beschränkungen von Äquivalenten zu den Ansprüchen zu betrachten sind, und dass andere Ausführungsformen verwendet und Änderungen vorgenommen werden können, ohne vom Umfang der beanspruchten Erfindung abzuweichen.

Unterschiedliche Ausführungsformen der Erfindung können weitere zweckmäßige Kombinationen der beschriebenen Elemente, Komponenten, Merkmale, Teile, Schritte, Mittel usw. umfassen, aufweisen, aus ihnen bestehen oder im Wesentlichen aus ihnen bestehen, auch wenn solche Kombinationen hier nicht spezifisch beschrieben sind. Darüber hinaus kann die Offenbarung andere Erfindungen umfassen, die gegenwärtig nicht beansprucht sind, die aber in Zukunft beansprucht werden können, insbesondere wenn sie vom Umfang der unabhängigen Ansprüche umfasst sind.

Erläuterungen, die sich auf Vorrichtungen, Apparate, Anordnungen, Systeme usw. gemäß Ausführungsformen der vorliegenden Erfindung beziehen, können auch für Verfahren, Prozesse, Methoden usw. gemäß den Ausführungsformen der vorliegenden Erfindung gelten und umgekehrt. Gleiche, gleich wirkende, in ihrer Funktion einander entsprechende, baulich identisch oder vergleichbar aufgebaute Elemente, Verfahrensschritte usw. können mit identischen Bezugszeichen angegeben sein.

Die nachfolgenden Erläuterungen und Definitionen, die einige Grundlagen der Erfindung betreffen, können für alle oder einen Teil der hier vorgestellten Ausgestaltungen gelten, und die Erläuterung bestimmter Aspekte im Zusammenhang mit nur einem Teil oder einer der Ausgestaltungen soll nicht dahingehend verstanden werden, dass diese Aspekte nicht auch mit anderen oder allen Ausgestaltungen, soweit technisch möglich und sinnvoll, verwirklicht sein können.

Sämtliche hier verwendeten Prozentangaben können sich auf Mol-, Mengen- oder Volumenanteile beziehen. Druckangaben in bar sind, soweit nicht anders erläutert, insbesondere als Absolutdrücke zu verstehen.

Die Konjunktion "und/oder" soll, wenn in einer Aufzählung vor dem letzten Begriff der Aufzählung verwendet, so verstanden werden, dass alle in der Aufzählung zuvor genannten Begriffe in beliebiger Weise miteinander kombiniert werden können. Mit anderen Worten ist mit "A, B und/oder C" "A und/oder B und/oder C" oder "wenigstens eines der Elemente A, B und C in beliebiger Kombination" gemeint.

Ist vorliegend von einem "Teil" eines Stoffstroms die Rede, kann es sich hierbei um einen Mengenanteil mit gleicher Zusammensetzung handeln, der von einem Ausgangsstrom lediglich abgezweigt wurde, aber auch um einen Anteil abweichender Zusammensetzung und ggf. nur eine Komponente des Ausgangsstroms, der mittels eines Verfahrens wie Kondensation, Evaporation, Sieden, Destillieren, Rektifizieren, Absorbieren, Adsorbieren, Flashen, Membrantrennen, Abscheiden oder dergleichen gebildet wird oder bei einem entsprechenden Schritt als Rest verbleibt. Ein "Teil" kann auch nach einer Kombination beliebiger der vorstehend benannten Schritte vorliegen, beispielsweise nach trenntechnischer Bearbeitung abgezweigten Anteils.

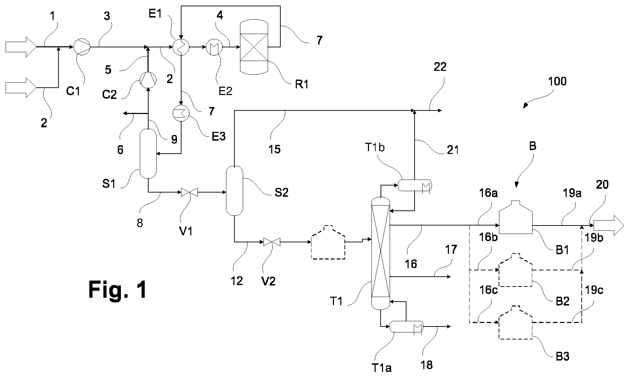

In Figur 1 sind Aspekte einer Methanolsynthese in Form eines vereinfachten Prozessflussdiagramms eines Verfahrens 100 veranschaulicht. Hierbei wird ein Frischeinsatzgemisch 3 bereitgestellt, das im hier veranschaulichten Fall insbesondere unter Verwendung von Wasserstoff 1, beispielsweise aus einer Elektrolyse, und Kohlenstoffdioxid 2, beispielsweise aus einer Kohlendioxidrückgewinnung, bereitgestellt wird. Die in Figur 1 veranschaulichte Methanolsynthese eignet sich also insbesondere zur Bereitstellung von Methanol aus zumindest teilweise nichtfossilen Quellen bzw. unter Verwendung von Kohlenstoffdioxid, das aus Rauchgas oder anderen Gasgemischen abgetrennt wird.

In der in Figur 1 veranschaulichten Methanolsynthese wird ein entsprechendes Frischeinsatzgemisch 3 typischerweise also nicht in Form eines herkömmlichen Synthesegases bereitgestellt, wie es durch Dampfreformierung, autotherme Reformierung oder partielle Oxidation aus fossilen Rohstoffen in einem kontinuierlichen Prozess gewonnen werden kann. Gleichwohl kann auch dies in Ausgestaltungen der Erfindung zusätzlich und/oder zeitweise erfolgen. Beliebige Mischungen der für die Methanolsynthese erforderlichen Komponenten Wasserstoff 1, Kohlenstoffdioxid 2 und/oder Kohlenstoffmonoxid können in beliebiger Weise, zu unterschiedlichen Zeiten in unterschiedlicher Weise, und in beliebigen Anteilen bereitgestellt werden.

Die Umsetzung von Kohlenstoffdioxid 2 zu Methanol kann in Konstellationen, in denen entsprechendes Kohlenstoffdioxid zur Verfügung steht, grundsätzlich auch eine Umwandlung von Kohlenstoffdioxid 2 zu Kohlenstoffmonoxid in einer reversen bzw. umgekehrten Wassergasshiftreaktion oder eine Kohlenstoffdioxidelektrolyse umfassen. Die "direkte" Hydrierung von Kohlenstoffdioxid 2 ist jedoch aufgrund ihrer geringeren Prozesskomplexität von Vorteil. Außerdem ist dieser Weg aufgrund der geringeren Kohlenstoffmonoxidkonzentration bzw. der Abwesenheit von Kohlenstoffmonoxid mit einer geringeren Bildung von Nebenprodukten verbunden.

Die wichtigsten Reaktionen bei der Methanolsynthese sind die eigentliche Methanolbildung und eine Shiftreaktion, die in den folgenden Reaktionsgleichungen angegeben sind. Die Methanolbildung ist dabei, wie dargestellt, aus Wasserstoff 1 und Kohlenstoffmonoxid oder durch die erwähnte "direkte" Hydrierung von Kohlenstoffdioxid 2 möglich.

Methanolbildung:

| | |
|---|---|
| CO + 2 H₂ ↔ CH₃OH | Δ H_{R} = - 90,77 kJ/mol |
| CO₂ + 3 H₂ ↔ CH₃OH + H₂O | Δ H_{R} = - 49,16 kJ/mol |

Shiftreaktion:

| | |
|---|---|
| CO₂ + 2 H₂ ↔ CO + H₂O | Δ H_{R} = - 41,21 kJ/mol |

In dem in Figur 1 veranschaulichten Fall kann durch die Einstellung der Mengen an Wasserstoff 1 und Kohlenstoffdioxid 2 insbesondere eine geeignete Stöchiometriezahl des Frischeinsatzgemischs 3 eingestellt werden. Der Begriff der Stöchiometriezahl wird vorliegend in der fachüblichen Weise verwendet. Beispielsweise wird die Stöchiometriezahl im Zusammenhang mit der Synthese von Methanol und Dimethylether in einem Artikel von I. Kiendl et al., "Experimentelle und theoretische Betrachtungen der Methanol- und direkten DME-Synthese im Labor- und Technikums-Maßstab", Chem. Ing. Tech. 2020, 92, No. 6, 736-745, erläutert.

In einem Durchgang kann in einer Methanolsynthese typischerweise eine Kohlenstoffumwandlung von nur 50% bis 80% erreicht werden, abhängig von der Einsatzzusammensetzung, den Prozessbedingungen, dem Katalysator und dem gewählten Verfahren. Daher wird zumindest ein Teil der nicht umgesetzten Komponenten, insbesondere abzüglich eines Purge- oder Spülgasanteils, in die Methanolsynthese zurückgeführt, um die Kohlenstoffumwandlung auf diese Weise zu steigern. Es wird also ein Methanolsynthesekreislauf geschaffen.

Ein entsprechend rückgeführtes Gasgemisch ist in Figur 1 mit 5 angegeben. Es wird nachfolgend, der besseren Unterscheidung von dem Frischeinsatzgemisch 3 halber, auch als Rückführgemisch 5 bezeichnet. Ein vorab abgezweigter Spülgasanteil ist mit 6 angegeben. Das Rückführgemisch 5 wird mittels eines Rückführgemischverdichters C2 verdichtet und mit dem mittels eines Frischeinsatzgemischverdichters C1 verdichteten Frischeinsatzgemisch 3 vereinigt. Nach der Vereinigung wird das nun als Reaktionseinsatzgemisch 4 bezeichnete Gasgemisch in einem Feed-Effluent-Wärmetauscher E1 und dann in einem weiteren Wärmetauscher E2 bzw. Heizer erwärmt und, weiter mit 4 bezeichnet, einem Methanolreaktor R1 zugeführt.

Die exotherme Bildung von Methanol findet im Methanolreaktor R1 bei beliebigen und die Erfindung nicht einschränkenden Reaktionsbedingungen statt. Abwärme kann beispielsweise zur Herstellung von Sattdampf aus Kesselspeisewasser genutzt werden. Heißes Prozessgas 7 aus dem Methanolreaktor R1 wird zur Erwärmung des Reaktionseinsatzgemischs 4 in dem Feed-Effluent-Wärmetauscher E1 verwendet. Das weiter mit 7 bezeichnete Prozessgas wird in einem Wärmetauscher E3 weiter abgekühlt, nachdem es den Feed-Effluent-Wärmetauscher E1 passiert hat. Kondensationswärme von Methanol und Wasser kann an einem anderen Punkt des Prozesses genutzt werden.

Der Methanolreaktor R1 kann in fachüblicher Weise als Einzelreaktor oder Reaktorsystem ausgebildet sein, beispielsweise umfassend adiabatische Teilreaktoren mit Zwischenkühlern, quenchgekühlten (Teil-)Reaktoren, gasgekühlten (Teil-) Reaktoren und isotherme, dampfproduzierende (Teil-)Reaktoren in beliebiger serieller und/oder paralleler Anordnung. Ausgestaltungen der Erfindung sind durch die Art des oder der Methanolreaktoren R1 in keiner Weise beschränkt.

Rohmethanol 8 in dem Prozessgas 7 wird in einem Separator S1 von einer Gasphase 9 abgetrennt, wobei die Gasphase 9 zur Bereitstellung des Rückführgemischs 5 und des Spülgasanteils 6 verwendet wird. Das Rohmethanol 8 wird über ein Ventil V1 in einen zweiten Separator S2 eingespeist, wo leichte Komponenten 15 aus dem Rohmethanol 8 ausgasen bzw. ausgetrieben werden, bevor dieses, nun mit 12 bezeichnet, einer Destillationskolonne T1 mit einem Sumpfverdampfer T1a und einem Kopfkondensator T1b zugeführt wird. Zur Entkopplung der Methanolsynthese und der Aufarbeitung des Rohmethanols 12 unter Verwendung der Destillationskolonne T1 kann optional ein Rohmethanolspeicher B4 vorgesehen sein.

Klassische Syntheseprozesse für Methanol aus Synthesegas werden mit geringen Laständerungsgradienten konstant betrieben. Für die Synthese von Methanol unter Einsatz einer direkten Hydrierung, bei der, wie erwähnt, im Wesentlichen Wasserstoff 1 und Kohlenstoffdioxid 2 in einem entsprechenden Frischeinsatzgemisch 3 eingesetzt werden, ändern sich im Vergleich zu einem klassischen, auf fossilem Synthesegas basierenden Prozess, wo sich auch Kohlenstoffmonoxid im Synthesegas findet, die Bedingungen des Methanolprozesses. Dies ist insbesondere durch die geringere exothermen Reaktionswärme (siehe oben zu Reaktionsenthalpien), den höheren Wassergehalt im Reaktionsprodukt, den geringeren Gehalt an Inertstoffen (der hauptsächlich abhängig ist von der Reinheit des Kohlenstoffdioxids), der langsameren Reaktionskinetik, der höheren Menge an Katalysator, die für dieselben Prozessbedingungen erforderlich ist, und der höheren Lastwechselgeschwindigkeit und der steileren Rampen bedingt.

Für weitere Details entsprechender Verfahren sei auf einschlägige Fachliteratur wie beispielsweise den Artikel "Methanol" in Ullmann's Encyclopedia of Industrial Chemistry vom 15 October 2012 und das Fachbuch von A. Tremel, "Electricity-based Fuels", Springer-Verlag 2018, verwiesen.

Wenn der ein entsprechender Methanolsynthesekreislauf mit abgeschiedenem Kohlenstoffdioxid 2 aus anderen Prozessen und Wasserstoff, der durch Elektrolyse aus erneuerbarem, kostengünstigem Strom (wie Photovoltaik und/oder Wind) erzeugt wird, muss ein entsprechender Methanolsynthesekreislauf insbesondere an ein stark schwankendes Produktionsprofil von Wasserstoff 1 angepasst werden. Kohlenstoffdioxid 2 kann in der Regel einfacher entsprechend des jeweiligen Bedarfs zugeführt werden. Insbesondere existieren Zeiten, beispielsweise nachts oder bei Flauten, in denen wenig oder kein Wasserstoff 1 durch Elektrolyse erzeugt werden kann. Hier vorgeschlagene Ausgestaltungen sehen daher insbesondere die erwähnte Zwischenspeicherung von Rohmethanol in dem Rohmethanolspeicher B4 vor.

Das Rohmethanol wird bei der herkömmlichen Herstellung von Methanol aus kohlenstoffmonoxidhaltigem Synthesegas normalerweise in einer, zwei oder bis zu drei Destillationskolonnen gereinigt. Die Verwendung einer einzigen Destillationskolonne T1, wie in Figur 1 veranschaulicht, ist insbesondere dann möglich, wenn die direkte Hydrierung von Kohlenstoffdioxid 2 eingesetzt wird und der Einsatz eines Strippers bzw. des erwähnten Separators S2 zur Entfernung leichter Verbindungen erfolgt. Die Reinigung bei der Direkthydrierung weicht leicht von der im herkömmlichen Prozess ab und kann aufgrund der erwähnten erhöhten Wasserproduktion im Methanolreaktor R1 energieintensiver sein. Jedoch ist die Reinigung bei beiden Verfahrensvarianten ausgesprochen energieintensiv und weist eine geringe Dynamik auf.

Typische Probleme, die bei niedrigen Lasten in einer Destillationskolonne 10 auftreten können, sind dem Fachmann bekannt und in der Fachliteratur wie beispielsweise bei F. Zhu et al., "Distillation Column Operating Window", in: "Hydroprocessing for Clean Energy: Design, Operation, and Optimization", Wiley-VCH, 2016, erläutert. Es handelt sich insbesondere um das sogenannte Weeping und Coning bzw. Spraying.

Ausgestaltungen der Erfindung nutzen insbesondere aus, dass sich die Einsatzgebiete für Methanol in der letzten Zeit geändert haben. Neben der Weiterverarbeitung in katalytischen Prozessen, für die hochreines Methanol benötigt wird, wurde Methanol als Energieträger, beispielsweise als Schiffskraftstoff, vorgeschlagen. Für diese Anwendung gelten andere Reinheitsanforderungen, die tendenziell niedriger sind als jene bei katalytischen Prozessen.

Destillationskolonnen, wie sie für die Reinigung von Methanol verwendet werden, weisen eine geringe Dynamik auf, was die Gesamtdynamik des Prozesses verlangsamt und möglicherweise den Lastbereich des Prozesses einschränkt. Die grüne Methanolproduktion leidet unter den hohen Energiepreisen in Zeiten geringer erneuerbarer Produktion und der geringeren Verfügbarkeit von Wärme (Dampf) an speziellen Produktionsstandorten. Der Bedarf an einem dynamischen Betrieb ist stark gestiegen. Die Dynamik der Destillationskolonnen und des Prozesses kann entkoppelt werden, indem Rohmethanol zwischengelagert wird und die Destillationskolonnen mit geringeren Schwankungen betrieben werden als der übrige Prozess. In diesem Fall ist jedoch immer noch weniger Wärme aus dem übrigen Prozess und/oder erneuerbare Energie verfügbar, um die Destillationskolonnen in Betrieb zu halten.

Für einige Verunreinigungen, wie Schwefelverbindungen und andere anorganische Stoffe, gelten auch bei den zwischenzeitlich entstandenen neuen Verwendungszwecken weiterhin strenge Grenzwerte. Allerdings sind diese Verunreinigungen bei grünen Methanolprozessen, d.h. insbesondere Methanolprozessen auf Basis von Wasserstoff 1 aus einer Elektrolyse und/oder Kohlenstoffdioxid 2 aus einer Abtrennung aus Rauchgas, aufgrund der verwendeten hochreinen Einsatzströme oder aufgrund strenger Katalysatoranforderungen (z.B. in Bezug auf Schwefel) oft von Natur aus nicht vorhanden.

Hier vorgeschlagene Ausgestaltungen bieten Lösungen zur Herstellung von Methanol, insbesondere in Kraftstoffqualität, bei schwankender Energieverfügbarkeit. Das vorgeschlagene Verfahren und seine Ausgestaltungen erzeugen insbesondere einen Methanolstrom mit schwankender Reinheit, hier auch als Methanolvorprodukt 16 bezeichnet, der neben einem Wasserstrom 18, einem Nebenproduktstrom 17, und einem Kopfgasstrom 21 der Destillationssäule T1 entnommen wird. Das Methanolvorprodukt 16 wird in einen oder mehrere Tanks B1, B2, B3 einer insgesamt mit B angegebenen Tankanordnung geleitet. Der Prozess wird dabei so gesteuert, dass das aus Tankanordnung B insgesamt entnommene Methanolprodukt 20 stets eine vorgegebene Reinheitsanforderung erfüllt, während die Reinheit des Methanolvorprodukts 16 bzw. in der Tankanordnung B gespeicherter Teilströme 16a, 16b, 16c hiervon vorübergehend gegen die Reinheitsanforderung verstoßen kann. Der Kopfgasstrom 21 kann mit den leichten Komponenten 15 aus dem Separator S2 zu einem Sammelstrom 22 vereinigt werden.

Im hier verwendeten Sprachgebrauch wird also Methanol und Wasser enthaltendes Rohmethanol 12 bereitgestellt und. Ferner wird ein gegenüber dem Rohmethanol 12 an Methanol angereichertes und Wasser abgereichertes Methanolzwischenprodukt 16 mit einem über die Zeit schwankenden Methanolgehalt unter Verwendung des Rohmethanols 12 oder eines Teils hiervon bereitgestellt. Es erfolgt ein Einspeisen des Methanolzwischenprodukts 16 oder eines Teils hiervon in eine Tankanordnung B. Ein Bereitstellen des Methanolprodukts 20 umfasst eine Entnahme des in der Tankanordnung B gespeicherten Methanolzwischenprodukts 16 oder eines Teils hiervon, wobei die Entnahme des in der Tankanordnung B gespeicherten Methanolzwischenprodukts 16 so gesteuert wird, dass das Methanolprodukt 20 einen Methanolgehalt in einem vorgegebenen Bereich aufweist.

Das hier vorgeschlagene Verfahren 100 und seine Ausgestaltungen ermöglichen eine vorteilhafte Herstellung von Methanol in Kraftstoffqualität bei schwankender Energieverfügbarkeit. Während heute der größte Teil des produzierten Methanols in katalytischen Prozessen u.a. zu Formaldehyd, Essigsäure und Methyl-tert-Butylether weiterverarbeitet wird, rückt die zusätzliche Verwendung von Methanol als erneuerbarer Kraftstoff und zur Speicherung erneuerbarer Energie in den industriellen Fokus. Diese Verwendung, insbesondere wenn die Endanwendung die Verbrennung des Methanols einschließt (z.B. in Schiffsmotoren), eröffnet die Möglichkeit, Methanol mit einem viel geringeren Reinheitsgrad zu produzieren, als dies heute üblich ist. Wenn dieses nicht mit anderen Kraftstoffen gemischt wird, kann Kraftstoff-Methanol beträchtliche Wasseranteile haben, z.B. 5% bei Verbrennungsmotoren, wodurch sich die Anzahl der erforderlichen Böden und/oder die Leistung des Sumpfverdampfers in den Kolonnen deutlich verringert.

Während die Reinheitsanforderungen für Kraftstoff-Methanol geringer sind als die für die konventionelle Methanolproduktion (z.B. mit Güteklasse A oder AA), sind die dynamischen Anforderungen des mit erneuerbarem Strom betriebenen Prozesses eine größere Herausforderung. Destillationskolonnen T1, wie sie üblicherweise für die Methanolreinigung verwendet werden, besitzen aufgrund der verwendeten Siebböden einen sehr begrenzten Beladungsbereich von beispielsweise 70% bis 110%.

In dem vorgeschlagenen Verfahren 100 kann in Zeiten geringer Energieverfügbarkeit die Methanolproduktion im Methanolreaktor R1 beispielsweise um mehr als 40%, insbesondere um mehr als 80%, reduziert oder sogar fast vollständig oder ganz eingestellt werden. Die Destillationssäule T1 kann auch in Zeiten vollständigen Stillstands des Methanolreaktors R1 durch die Verarbeitung der aus dem Rohmethanoltank B4 abgelassenen Flüssigkeit in Betrieb gehalten werden. Insbesondere kann die Destillationssäule T1 nahe dem unteren Ende ihres Betriebsfensters betrieben werden. Um Energie zu sparen, kann die Reinheit des Methanolvorprodukts 16 zu diesem Zeitpunkt reduziert werden.

Während einer Zeit hoher Energieverfügbarkeit kann Destillationssäule T1 am oberen Ende ihres Betriebsfensters betrieben werden. Um die Zeiten der Methanolproduktion mit geringer Reinheit zu kompensieren, wird die Destillationssäule T1 so betrieben, dass eine höhere Methanolreinheit erreicht wird als in der anderen Betriebsart.

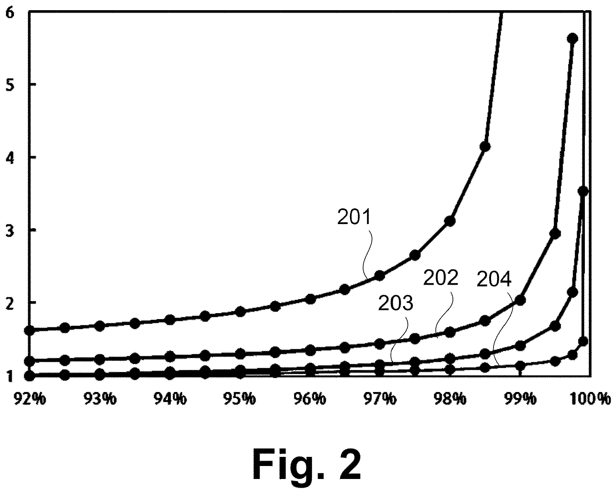

Figur 2 zeigt das Verhältnis zwischen dem Reinheitsbedarf und der spezifischen Reboilerleistung des Sumpfverdampfers T1a pro Einheit des produzierten Methanols. Die Werte beziehen sich auf eine Prozesskonfiguration, wie sie beispielsweise in dem Figur 1 veranschaulichten Verfahren 100 verwendet wird.

In Figur 2 ist dabei eine Methanolreinheit in Prozent auf der Horizontalachse gegenüber einem Verhältnis der Reboilerleistung zu einer minimalen Reboilerleistung des Sumpfverdampfers T1a dargestellt. Die Graphen 201 bis 204 beziehen sich dabei, in der angegebenen Reihenfolge und von oben nach unten in Figur 2, auf Destillationssäulen T1 mit 10, 12, 15 und 20 Trennstufen.

In Ausgestaltungen kann auch eine Bereitstellung von Tanks für Prozesswasser, insbesondere des Wasserstroms 18, vorgesehen sein, die eine vorübergehende Verletzung der Abwasserspezifikationen ermöglichen.

Ausgestaltungen der Erfindung können Regelungsverfahren wie die modellprädiktive Regelung (MPC), die nichtlineare MPC (NMPC) und die ökonomische NMPC (eNMPC) können eingesetzt werden, um die Destillationssäule T1 optimal zu betreiben und das produzierte Methanol auf verschiedene Produkttanks zu verteilen, falls vorhanden. Die modellprädiktive Regelung wird vorzugsweise mit einem Mittel zur Abschätzung der Energieverfügbarkeit und des Produktbedarfs in der Zukunft kombiniert.

Ein zusätzlicher Produkttank kann für Produkte, die aufgrund unzureichender Reinheit zurückgewiesen werden, vorgesehen sein. Es kann notwendig sein, Produkte auszusortieren, z.B. bei unerwarteten Ereignissen oder Laständerungen. Das in diesem Tank gelagerte Produkt kann für interne Prozesse verwendet werden, z.B. für die Wärmeversorgung der Destillationskolonnen, oder es kann in den Rohmethanoltank zurückgeführt werden.

Wenngleich zuvor bestimmte Merkmale hier vorgeschlagener Ausgestaltungen in bestimmter Kombination beschrieben wurden, können andere Ausgestaltungen auch abweichende Kombinationen umfassen, wobei grundsätzlich eine Beschränkung nur durch die Grenzen technischer Sinnhaftigkeit und Praktikabilität gegeben ist.

## Patentansprüche

1. Verfahren (100) zur Erzeugung eines Methanolprodukts (20), das ein Bereitstellen von Methanol und Wasser enthaltendem Rohmethanol (12), ein Bereitstellen eines gegenüber dem Rohmethanol (12) an Methanol angereicherten und Wasser abgereicherten Methanolzwischenprodukts (16) mit einem über die Zeit schwankenden Methanolgehalt unter Verwendung des Rohmethanols (12) oder eines Teils hiervon, ein Einspeisen des Methanolzwischenprodukts (16) oder eines Teils hiervon in eine Tankanordnung (B), und ein Bereitstellen des Methanolprodukts (20) umfassend eine Entnahme des in der Tankanordnung (B) gespeicherten Methanolzwischenprodukts (16) oder eines Teils hiervon umfasst, wobei die Entnahme des in der Tankanordnung (B) gespeicherten Methanolzwischenprodukts (16) derart vorgenommen wird, dass das Methanolprodukt (20) einen Methanolgehalt in einem Vorgabebereich aufweist.

2. Verfahren (100) nach Anspruch 1, bei dem die Entnahme des in der Tankanordnung (B) gespeicherten Methanolzwischenprodukts (16) dann vorgenommen wird, wenn sich in der Tankanordnung (B) aufgrund der Einspeisung ein Methanolgehalt in dem Vorgabebereich eingestellt hat.

3. Verfahren (100) nach Anspruch 1 oder 2, bei dem eine Tankanordnung (B) mit mehreren Methanolzwischenprodukttanks (B1, B2, B3) verwendet wird, wobei das Methanolzwischenprodukt (16) in Abhängigkeit von seinem Methanolgehalt in unterschiedliche der Methanolzwischenprodukttanks (B1, B2, B3) eingespeist wird.

4. Verfahren (100) nach Anspruch 3, bei dem das Bereitstellen des Methanolprodukts (20) eine Entnahme des in der Tankanordnung (B) gespeicherten Methanolzwischenprodukts (16) aus einem oder mehrerer der unterschiedlichen Methanolzwischenprodukttanks (B1, B2, B3) umfasst.

5. Verfahren (100) nach einem der vorstehenden Ansprüche, bei dem das Bereitstellen des Methanolzwischenprodukts (16) unter Verwendung einer Destillationssäule (T1) vorgenommen wird.

6. Verfahren (100) nach Anspruch 5, bei dem die Destillationssäule (T1) einen Sumpfverdampfer (T1a) aufweist, der mit einer über die Zeit schwankenden Heizleistung betrieben wird.

7. Verfahren (100) nach Anspruch 6, bei der die Heizleistung an eine Energieverfügbarkeit und/oder einen Energiepreis angepasst wird.

8. Verfahren (100) nach einem der vorstehenden Ansprüche, bei dem das Rohmethanol (12) einen Gehalt von 45 bis 55 mol-% Methanol, von 45 bis 55 mol-% Wasser und von 0 bis 2 mol-% weiterer Komponenten aufweist.

9. Verfahren (100) nach einem der vorstehenden Ansprüche, bei dem das Methanolvorprodukt (16) mit einem Gehalt von 80 bis 100 mol-% Methanol bereitgestellt wird und bei dem der Methanolgehalt des Methanolprodukts (20) bei 95 bis 98 mol-% liegt.

10. Verfahren (100) nach einem der vorstehenden Ansprüche, bei dem das Bereitstellen des Rohmethanols (12) ein Umsetzen von Wasserstoff (1) mit Kohlenstoffdioxid (2) umfasst.

11. Verfahren (100) nach Anspruch 10, bei dem das Rohmethanol in einem Rohmethanolspeicher (B4) zwischengespeichert wird.

12. Verfahren (100) nach einem der vorstehenden Ansprüche, das die Verwendung eines modellbasierten Regelungsverfahrens umfasst.

13. Verfahren (100) nach einem der vorstehenden Ansprüche, bei dem Prozesswasser (18) mit einem über die Zeit unterschiedlichen Gehalt von anderen Komponenten bereitgestellt und zeitweise zwischengespeichert wird.

14. Anlage zur Erzeugung eines Methanolprodukts (20), die zum Bereitstellen von Methanol und Wasser enthaltendem Rohmethanol (12), zum Bereitstellen eines gegenüber dem Rohmethanol (12) an Methanol angereicherten und Wasser abgereicherten Methanolzwischenprodukts (16) mit einem über die Zeit schwankenden Methanolgehalt unter Verwendung des Rohmethanols (12) oder eines Teils hiervon, zum Einspeisen des Methanolzwischenprodukts (16) oder eines Teils hiervon in eine Tankanordnung (B), und zum Bereitstellen des Methanolprodukts (20) umfassend eine Entnahme des in der Tankanordnung (B) gespeicherten Methanolzwischenprodukts (16) oder eines Teils hiervon eingerichtet ist, wobei die Anlage ferner dazu eingerichtet ist, die Entnahme des in der Tankanordnung (B) gespeicherten Methanolzwischenprodukts (16) derart vorzunehmen, dass das Methanolprodukt (20) einen Methanolgehalt in einem Vorgabebereich aufweist.

15. Anlage nach Anspruch 14, die zur Durchführung eines Verfahrens (100) nach einem der Ansprüche 1 bis 13 eingerichtet ist.
